**Europäisches Patentamt**

**European Patent Office**

**Office européen des brevets**

(19)

(11) Veröffentlichungsnummer: **0 063 814**
**B1**

(12)

# EUROPÄISCHE PATENTSCHRIFT

(45) Veröffentlichungstag der Patentschrift:
**05.12.84**

(21) Anmeldenummer: **82103531.8**

(22) Anmeldetag: **27.04.82**

(51) Int. Cl.³: **C 07 C 43/04,** C 07 C 41/06,
C 07 C 41/42, C 07 C 31/12,
C 07 C 29/04, C 07 C 29/86 //
C07C11/08, C07C11/09,
C07C11/167, C07C9/12,
C07C5/13, C07C5/22, C07C5/25,
C07C5/333

(54) Verfahren zur Herstellung von Gemischen aus sec.-Butyl-tert.-butylether und tert.-Butylalkohol.

(30) Priorität: **28.04.81 DE 3116780**

(43) Veröffentlichungstag der Anmeldung:
**03.11.82 Patentblatt 82/44**

(45) Bekanntmachung des Hinweises auf die Patenterteilung:
**05.12.84 Patentblatt 84/49**

(84) Benannte Vertragsstaaten:
**AT BE CH DE FR GB IT LI LU NL SE**

(56) Entgegenhaltungen:
**CA - A - 958 213**
**DE - A - 2 620 011**
**FR - A - 2 367 721**
**US - A - 3 849 082**

**L'INDUSTRIE DU PETROLE, Band 48, Nr. 524, 1980, Seiten 57-62, Paris, FR. G. JACQUES: "New petrochemical processes"**

**Die Akte enthält technische Angaben, die nach dem Eingang der Anmeldung eingereicht wurden und die nicht in dieser Patentschrift enthalten sind.**

(73) Patentinhaber: **VEBA OEL AG,**
**Alexander-von-Humboldt-Strasse,**
**D-4650 Gelsenkirchen-Hassel (DE)**

(72) Erfinder: **Gottlieb, Klaus, Dr., Alte Strasse 59,**
**D-5804 Herdecke-Ende (DE)**
Erfinder: **Graf, Wilfried, Lütkenbusch 4, D-4270 Dorsten (DE)**

(74) Vertreter: **Krug, Joachim, Dr.,**
**Alexander-von-Humboldt-Strasse,**
**D-4650 Gelsenkirchen-Hassel (DE)**

## Beschreibung

Die Erfindung betrifft ein Verfahren zur Herstellung von sec.-Butylalkohol und sec.-Butyl-tert.-butylether sowie tert.-Butylalkohol aus Butane enthaltenden Kohlenwasserstoffgemischen, wie sie z.B. bei der Rohölförderung oder der Rohölverarbeitung anfallen.

Das Sammeln, Reinigen, Transportieren und Verteilen dieser Butane enthaltenden Paraffinkohlenwasserstoffe ist sehr aufwendig und mit hohen Kosten verbunden. Die Verwendung des $C_4$-Anteiles dieser Paraffinkohlenwasserstoffe als Kraftstoff in Verbrennungsmaschinen ist, obwohl technisch möglich, aus genannten Gründen energetisch ungünstig. Die insgesamt für das Sammeln, Reinigen, Transportieren und Verteilen aufzuwendende Energie ist im Verhältnis zur in herkömmlichen Verbrennungsmotoren erzielten Energieausnutzung sehr gross, so dass langfristig der Einsatz der Butane als Autogas ohne steuerliche Begünstigung unwirtschaftlich ist.

Der vorliegenden Erfindung liegt die Aufgabe zugrunde, die Butane, die heute noch in grösseren Mengen abgefackelt werden mit höherer Energierentabilität in wertvolle Kraftstoffkomponenten umzuwandeln, deren Erzeugung, Transport und Verteilung einen geringeren Energieeinsatz verlangt als Sammeln, Reinigen, Transport und Verteilung der gasförmigen Kohlenwasserstoffe und deren Einsatz in herkömmlichen Verbrennungsmotoren ohne Zusatzausrüstung möglich ist und dort zur Verringerung des spezifischen und absoluten Kraftstoffverbrauchs sowie zur Umweltentlastung beiträgt.

Aus den DE-OSen 2 620 011 und 2 921 576 ist es bekannt, Butan zu Methyl-tert.-butylether zu verarbeiten. Hierbei wird n-Butan partiell oder vollständig zu Isobutan (2-Methyl-propan) isomerisiert und das n-Butan-Isobutan-Gemisch dehydriert, wobei neben Isobuten auch n-Butene, Butadien, Propen und Propan gebildet werden. Das Dehydrierungsreaktionsgemisch wird dann mit einem Überschuss an Methylalkohol veräthert, wobei sich das in der Dehydrierungsstufe gebildete Isobuten zu Methyl-tert.-butylether umsetzt. Der überschüssige Methylalkohol aus dem Verätherungsreaktionsgemisch wird entweder mit Wasser oder durch Azeotrop-Destillation entfernt. In der Isomerisierung und Dehydrierung von 25 bis zu 30 Gew.-% der eingesetzten Butanfraktion zu n-Buten, Butadien, Propan, Propen, Äthan, Äthen, Methan und Wasserstoff umgewandelt. Diese Isomerisier- und Dehydrierabgase können in den beschriebenen Verfahren nur als Brennstoff für Heizzwecke Verwendung finden, werden somit also erheblich abgewertet.

Hauptzweck des in OS 2 921 576 beschriebenen Verfahrens ist die Erzeugung von reinem Methyl-tert.-butylether bzw. des in OS 2 620 011 beschriebenen Verfahrens die Herstellung von reinem Methyl-tert.-butylether oder reinem Methyl-tert.-amylether, bzw. einem Gemisch dieser beiden reinen Äther aus Methylalkohol und tert. Olefinen.

Im Gegensatz hierzu ist das Ziel vorliegender Erfindung, nicht Reinkomponenten, sondern Gemische aus gegebenenfalls sec.-Butyl-Alkohol enthaltenden sec.-Butyl-tert.-butylether-tert.-Butylalkohol-Gemisch zu erzeugen, das in bestimmten Zusammensetzungen synergistische Effekte bei der Verbrennung im Motor zeigt und die, wie Experimente und Berechnungen zur Energienutzung aller eingesetzten und erzeugten Stoffe gezeigt haben, hinsichtlich des Gesamtenergieverbrauchs optimiert sind. Hierzu soll nach der vorliegenden Erfindung die Umwandlung auch des bei der Dehydrierung anfallenden n-Butens und Butadiens zu $C_4$-Alkohol und mit Isobuten zu dem auf $C_4$-Alkohol aufbauenden Äther erfolgen.

Hierfür wird gemäss vorliegender Erfindung n-Butan partiell isomerisiert, katalytisch dehydriert, im Dehydrierungsreaktionsgemisch Butadien selektiv unter gleichzeitiger Umwandlung von Buten-1 zu Buten-2 hydriert und das im Dehydrierungsreaktionsgemisch nach der Selektivhydrierung enthaltene Isobuten mit rückgeführtem sec-Butylalkohol-Wassergemisch zu sec-Butyl-tert.-butylether und tert.-Butylalkohol umgesetzt. Das sec.-Butylalkohol-Wassergemisch wird hergestellt durch Umsetzung von Wasser mit dem Buten-2 des vorwiegend Buten-2 und Butan enthaltenden Kohlenwasserstoffgemisches nach der Verätherung.

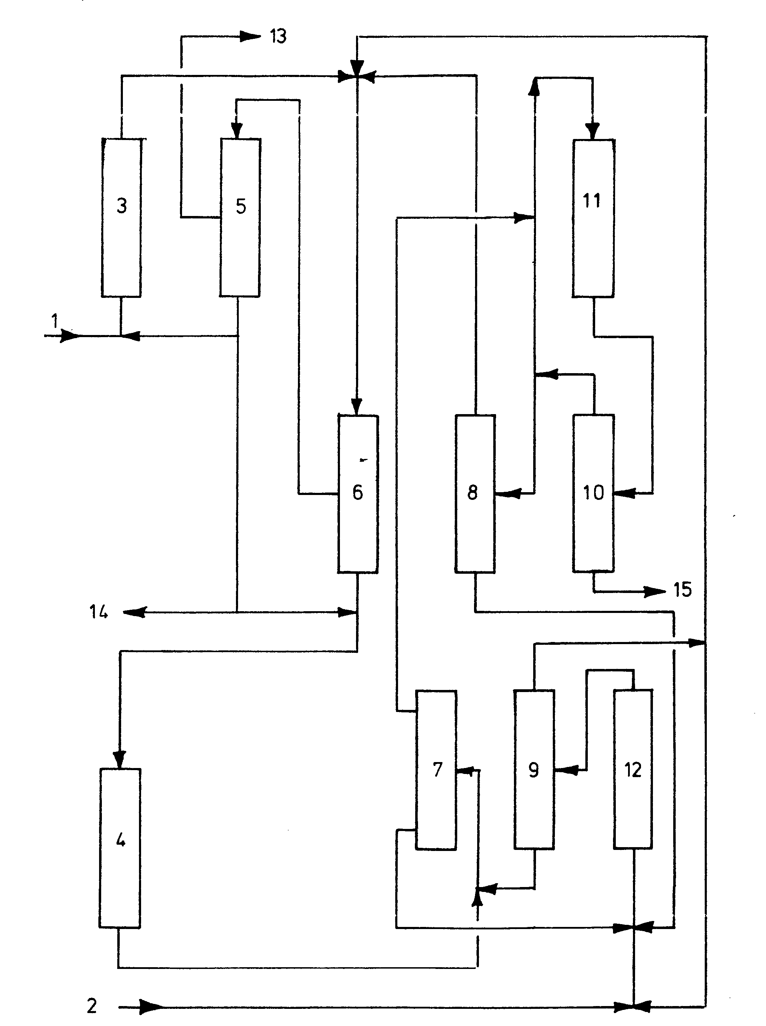

Weitere Ausbildungen der Erfindung ergeben sich aus der nachfolgenden Verfahrensbeschreibung in der das Verfahren unter Bezugnahme auf die in der zugehörigen Zeichnung enthaltene Prinzipskizze näher erläutert wird. Die Zeichnung stellt eine bevorzugte Ausführungsform des erfindungsgemässen Verfahrens unter Weglassung der für das Verständnis nicht erforderlichen Teile, wie Pumpen, Wärmetauscher und einiger Destillationskolonnen dar.

Gegebenenfalls Isobutan enthaltendes n-Butan 1, z.B. eine Butanfraktion der bei der Gewinnung oder Verarbeitung von Erdöl anfallenden Gase, wird einem Isomerisierungsreaktor 3 zugeführt. Die Isomerisierung — n-Butan zu Isobutan — erfolgt in an sich bekannter Weise an einem Platin-haltigen Festbettkatalysator in Gegenwart von Wasserstoff bei Temperaturen von 150-210 °C und Drücken von 15 bis 30 bar. Die Reaktionsbedingungen hinsichtlich Druck und Temperatur werden insgesamt so eingestellt, dass das Isomerisierungsgleichgewicht möglichst erreicht wird. Von dem den Isomerisierungsreaktor 3 verlassenden, zu etwa 50 Gew.-% aus Isobutan bestehenden Reaktionsgemisch werden der Wasserstoff, bei der Isomerisierung entstandenes Methan, Äthan und Propan abgetrennt. Das Isomerisat wird zusammen mit dem rückgeführten Isobutan aus der Verätherung und den rückgeführten Kohlenwasserstoffen aus der Hydratisierung der Dehydrierung 6 zugeführt.

Durch die Isomerisierung wird n-Butan in einer solchen Menge in Isobutan überführt, wie als vorbestimmte stöchiometrische Menge für die Verätherung der insgesamt erzeugten Alkohol-Wassermenge notwendig ist. Der Isobutangehalt des Butanstroms nach Isomerisierung beträgt 40-55 Gew.-%.

Die Dehydrierung 6 der $C_4$-Kohlenwasserstoffe erfolgt in an sich bekannter Weise katalytisch, wahlweise in einem Festbett- oder einem Fliessbettreaktor. Die Dehydrierungstemperatur liegt zwischen 530 und 700 °C, der Druck zwischen 0,2 und 5 bar, vorzugsweise zwischen 0,3 und 1,5 bar. Der Dehydrierungskatalysator besteht im allgemeinen aus aktivem Aluminiumoxid und Zusätzen aus Chromoxid oder Platin, die durch Tränken auf $Al_2O_3$ aufgebracht werden. Der während der Reaktionsphase entstehende Koks wird in einer Regenerierphase mit Luft abgebrannt, die dabei freiwerdende Wärme wird als Prozesswärme zurückgewonnen. Das Dehydrierungsreaktionsgemisch wird durch Abkühlen und Komprimieren in einen gasförmigen, vorwiegend leichtere Kohlenwasserstoffe und den Wasserstoff enthaltenden Strom und einen flüssigen, die Butane, Butadien und Butene enthaltenden Strom aufgetrennt.

Aus dem gasförmigen Strom wird der Wasserstoff in einer Reinigungsanlage 5 in an sich bekannter Weise weitgehend abgetrennt. Wenn keine adäquate Verwendungsmöglichkeit für den Gesamtwasserstoff besteht, wird der leichten Fraktion des Dehydrierungsgemisches nur so viel Wasserstoff entnommen, wie für die Isomerisierungs- und Hydrierungsreaktionen notwendig ist. Der Restwasserstoff kann bei 14 das Dehydrierabgas bei 13 zur Prozessenergieerzeugung entnommen werden. Der alle Kohlenwasserstoffe enthaltende Strom wird einer Selektivhydrierung und Hydroisomerisierung 4 zugeführt, wodurch Butadien selektiv zu Buten hydriert und gleichzeitig alles Buten-1 in Buten-2 überführt wird.

Die Selektivhydrierung und Hydroisomerisierung erfolgt in an sich bekannter Weise, katalytisch in Gegenwart von Wasserstoff in einem Festbettreaktor. Die Temperatur beträgt 20-80°C, vorzugsweise 30 bis 60°C, der Druck 1-20 bar, vorzugsweise 1,5-10 bar. Der verwendete Katalysator besteht im allgemeinen aus einem Träger, z.B. Aluminiumoxid oder Siliziumoxid und Zusätzen aus Platin, Palladium oder Nickel.

Die Wasserstoffkonzentration und die Beschickungsgeschwindigkeit werden so gewählt, dass Butadien nahezu vollständig (Restgehalt an Butadien im Reaktionsendgemisch kleiner 0,5 Gew.-%) und Buten-1 in maximaler Ausbeute nahe dem thermodynamischen Gleichgewichtswert so in Buten-2 überführt werden, dass Butene in möglichst geringer Menge (weniger als 10 Gew.-%) zu n-Butan hydriert werden.

Zweck dieser Stufe ist es, nach der Verätherung Isobutan destillativ so abtrennen zu können, dass Butene und n-Butan im Sumpf der Kolonne 8 verbleiben. Die Siedelücke zwischen Isobutan einerseits, n-Butan und Buten andererseits ist so gross, dass eine einfache destillative Abtrennung des Isobutans möglich ist, wenn zuvor durch Hydroisomerisierung Buten-1 in Buten-2 überführt und Isobuten durch Verätherung abgetrennt wurde. In einer besonderen Ausführung des erfindungsgemässen Verfahrens, wird die Selektivhydrierung und Hydroisomerisierung nach der Verätherung angeordnet; dies ist dann vorzuziehen, wenn aufgrund der für die Verätherung gewählten Verfahrensbedingungen, die in diesem Falle nur in geringen Mengen aus Butadien sich bildenden polymeren Stoffe sich bei der Verätherung nicht störend bemerkbar machen.

Die Isobuten-haltige $C_4$-Fraktion wird mit einem sec.-Butylalkohol-Wasser-Gemisch in Gegenwart saurer Katalysatoren umgesetzt, wobei sec-Butylalkohol zu 10-95%, vorzugsweise zu 50-90% unter Bildung von sec.-Butyl-tert.-butylether und Wasser zu 50-100%, unter Bildung von tert.-Butylalkohol umgesetzt werden. Das als Beschickung verwendete sec.-Butylalkohol-Wasser-Gemisch kann 1-50 Gew.-% Wasser enthalten, insbesondere kann man bei einer Azeotrop-Destillation anfallendes sec.-Butylalkohol-Wasser-Gemisch einsetzen. Es wurde überraschenderweise gefunden, dass auch in Gegenwart von Wasser die n-Butene keine Umsetzung eingehen. Überraschenderweise wurde auch gefunden, dass tert.-Butylalkohol kein Reaktionsprodukt durch Parallelreaktion mit Isobuten bildet. Als Katalysator können sulfonierte stark saure Ionenaustauscher auf der Basis von sulfonierten, mit Divinylbenzol vernetztem Styrol verwendet werden. Die Umsetzung erfolgt in einem mehrstufigen Festbettreaktor bei Temperaturen zwischen 20 und 150°C, vorzugsweise bei 30-80°C und Drücken von 4-40 bar, vorzugsweise 8-16 bar. Das Molverhältnis von sec.-Butylalkohol zu Isobuten liegt im Bereich von 1:0,1 bis 1:10, vorzugsweise von 1:0,1 bis 1:5, das Molverhältnis von Wasser zu Isobuten im Bereich von 1:1 bis 1:20, vorzugsweise von 1:1,5 bis 1:10, die Raumgeschwindigkeit in Liter Beschickungsprodukt pro Liter Katalysator und Stunde im Bereich von 0,3 bis 50, vorzugsweise 1-20. Das Äther-Alkohol-Gemisch wird durch Destillation unter Druck von den nicht umgesetzten Kohlenwasserstoffen abgetrennt und bei 15 abgezogen. Die nicht umgesetzten Kohlenwasserstoffe werden in die Verätherung zurückgeführt.

Aus der DE-OS 2 535 471 ist die Herstellung von sec.-Butyl-tert.-butylether an sich bekannt. Im Unterschied zu der dort beschriebenen Ausführungsform, die auf einen während der Reaktion vorhandenen Überschuss an sec.-Butylalkohol und damit notwendigerweise auf höhere Temperaturen abhebt, wird in der vorliegend beschriebenen bevorzugten Ausführungsform mit einem Überschuss an Isobuten und niedrigeren Temperaturen gearbeitet, wodurch ein höherer Alkohol-Umsatz erreicht wird und somit auf eine Abtrennung und Rückführung des nicht umgesetzten Alkohols verzichtet werden kann.

Ein weiterer Vorteil der Arbeitsweise mit niedrigen Reaktionstemperaturen ist, dass eine Dehydratisierung des sec.-Butylalkohols zu Wasser und n-Buten-2 vermieden wird, was die Wirtschaftlichkeit des Verfahrens bei höheren Reaktionstemperaturen nachteilig beeinflusst.

Es wurde überraschenderweise gefunden, dass auch in Gegenwart von Wasser die n-Butene keine Umsetzung eingehen. Überraschenderweise wurde weiterhin festgestellt, dass tert.-Butylalkohol kein Reaktionsprodukt durch Parallelreaktion mit Isobuten bildet.

Die an sich bekannte Herstellung von tert.-Butylalkohol aus Isobuten und Wasser an sauren Ionenaustauschern ist zusammen mit der Herstellung von Äthern aus Isobuten und Alkohol im gleichen Reaktionsschritt bisher noch nicht beschrieben. Der besondere Wert dieses Verfahrens beruht darauf, dass bei sehr niedrigen Temperaturen und einem auf Wasser bezogenen Isobutenüberschuss ein nahezu 100- prozentiger Umsatz von Wasser zu tert.-Butylalkohol, ohne Bildung nennenswerter Mengen an Isobutenoligomeren erzielt wird. In den vorbeschriebenen Verfahren zur Herstellung von tert.-Butylalkohol muss dagegen mit einem auf Isobuten bezogenen Überschuss an Wasser gearbeitet werden, um den Zwangsfall an Isobutenoligomeren niedrig zu halten. Um eine ausreichende Ausbeute an tert.-Butylalkohol zu erreichen, wird auch bei hohen Temperaturen gearbeitet. Die Herstellung von wasserfreiem tert.-Butylalkohol nach den bekannten Verfahren ist zudem mit hohen Trennkosten verbunden.

Die nach der Verätherung abgetrennte, nur Isobutan, n-Butan und n-Butene enthaltende $C_4$-Fraktion wird einer Druckdestillatonskolonne 8 zugeführt, wo Isobutan von den restlichen $C_4$-Kohlenwasserstoffen abgetrennt wird. Isobutan wird in die Dehydrierung 6 zurückgeführt, die im Sumpf abgenommene Butenfraktion wird der Buten-Hydratisierung 12 zugeführt, wo durch katalytische Synthese von Buten und Wasser bei einem Druck von 20-80 bar und 100 bis 170°C, vorzugsweise bei 30-60 bar und 120 bis 160°C sec.-Butylalkohol hergestellt wird. Als Katalysatoren werden stark saure Ionenaustauscher verwendet, am meisten bevorzugt sulfonierte, mit Divinylbenzol vernetzte Polystyrolharze. Im Beschickungsstrom werden auf 1 Mol Buten 2-10 Mole Wasser, vorzugsweise 3-6 Mole Wasser eingesetzt. Die Raumgeschwindigkeit in Liter Beschickung pro Liter Katalysator und Stunde beträgt 0,2-15, vorzugsweise 0,5-5. Unter diesen Reaktionsbedingungen werden 5-35% des eingesetzten n-Butens unter Bildung von sec.-Butylalkohol und Spuren an Di-sec.-butylether umgesetzt. Von dem Reaktionsendgemisch werden durch einfaches Destillieren die $C_4$-Kohlenwasserstoffe über Kopf der Kolonne 9 abgetrennt und ein Teilstrom in den Hydratisierungsreaktor 12 zurückgeführt, ein mengenmässig kleiner Teilstrom wird in die Dehydrierung 6 rückgeführt. Das sec.-Butylalkohol-Wasser-Gemisch wird gegebenenfalls nach destillativer Anreicherung von sec.-Butylalkohol, mit einem als Extraktionsmittel für sec.-Butylalkohol geeigneten, mit Wasser nicht mischbaren und von sec.-Butylalkohol leicht abtrennbaren organischen Lösungsmittel vermischt. Gemäss einer besonderen Ausbildung dieser Erfindung wird dafür einer der im Verfahren erzeugten n-Buten- oder Isobuten enthaltenden $C_4$-Ströme verwendet. Nach Trennen des Extraktionsgemisches in eine organische Phase und eine wässrige Phase enthält die organische Phase 50-98% der entstandenen sec.-Butylalkoholmenge und 90-98% der Di-sec.-butylethermenge. Von der organischen Phase werden durch Destillation die $C_4$-Kohlenwasserstoffe abgetrennt und in die Extraktionsstufe 7 zurückgeführt. Das aus dem Sumpf der Destillation entnommene sec.-Butylalkohol-Wassergemisch wird einschliesslich des gebildeten Di-sec.-butylethers der Verätherung zugeführt.

In der bevorzugten Ausführungsform wird zur Extraktion 7 die Isobuten enthaltende $C_4$-Fraktion eingesetzt. Dazu werden 1 Gewichtsteil des aus dem Sumpf der Kolonne 9 abgezogenen Wasser-sec.-Butylalkohol-Gemisches mit 2-10 Gewichtsteilen der $C_4$-Fraktion vermischt und der Extraktionsstufe 7 zugeführt, wo das Gesamtgemisch in eine wässrige und eine organische Phase aufgetrennt wird. Die organische Phase enthält 50-60 Gew.-% des der Extraktion zugeführten sec.-Butylalkohols und Wasser. Durch Destillieren wird ein die gewünschte Menge Wasser enthaltendes Gemisch aus Wasser, sec.-Butylalkohol und Isobuten enthaltender $C_4$-Fraktion abgetrennt, das Isobuten, Wasser und sec.-Butylalkohol in der für die Verätherung notwendigen stöchiometrischen Menge enthält. Die in der Extraktion abgetrennte wässrige Phase wird in die Hydratisierung 12 zurückgeführt.

Um die Trennleistung der Extraktionsstufe zu erhöhen, kann aus dem, vom Sumpf der Kolonne 9 abgezogenen sec.-Butylalkohol-Wasser-Gemisch durch Destillieren zunächst ein an sec.-Butylalkohol angereichertes sec-Butylalkohol-Wasser-Gemisch abgetrennt und, wie vorgehend beschrieben, mit der Isobuten enthaltenden $C_4$-Fraktion behandelt werden. Der Anreicherungsgrad kann bis zu 80 Gew.-% betragen. Zur extraktiven Abtrennung des ver Verätherung zuzuführenden sec.-Butylalkohol-Wasser-Gemisches werden dann 1 Gewichtsteil des an sec.-Butylalkohol angereicherten wässrigen Gemisches mit 0,5-5 Gewichtsteilen Isobuten enthaltender $C_4$-Fraktion gemischt und der Extraktionsstufe 7 zugeführt, wo eine 80-98 Gew.-% des in der Hydratisierung gebildeten sec.-Butylalkohols und Wasser enthaltende organische Phase abgetrennt wird. Restwasser wird wieder in die Hydratisierung zurückgeführt. Anstelle der Isobuten enthaltenden $C_4$-Fraktion kann als Extrationsmittel auch die von Isobuten befreite, Buten-2 und n-Butan enthaltende $C_4$-Fraktion aus dem Einsatz zur Buten-Hydratisierung 12 verwendet werden.

Schliesslich kann in einer besonderen Ausführungsform des erfindungsgemässen Verfahrens ein nach der Hydratisierung 12 über Kopf einer Anreicherungskolonne abgenommenes sec.-Butylalkohol-Wasser-Gemisch direkt der Verätherung 11 zugeführt und das erfindungsgemässe tert.-Butylalkohol-haltige Äther-Alkohol-Gemisch hergestellt werden, das bei 15 abgezogen wird.

*Beispiel 1*

Ein 0,46 Mol Wasser und 0,64 Mol sec.-Butylalkohol enthaltendes Gemisch mit Isobuten im molaren Mengenverhältnis Wasser : sec.-Butylalkohol : Isobuten = 0,46 : 0,64 : 0,96 wird bei einer Temperatur von 60°C und einem Druck über dem Dampfdruck von Isobuten, so dass dieses als Flüssigkeit vorliegt, nämlich 16 bar, umgesetzt. Als Reaktor wurde ein schlanker Rohrreaktor mit einem Verhältnis von Innendurchmesser zu Länge von 1 : 30 und als Katalysator ein stark saures Ionenaustauscherharz (Handelsprodukt Amberlyst 15) verwendet. Der mit Katalysator gefüllte Reaktor wurde mit 4,4 Gewichtsteilen des genannten Wasser-sec.-Butylalkohol-Isobuten-Gemisches pro Stunde und pro Gewichtsteil getrockneten Katalysator beschickt. Zur Einstellung der genannten Temperatur wurde ein geeigneter Vorheizer verwendet; die bei der Reaktion freiwerdende Wärme wurde über einen Kühler abgeführt. Das Reaktionsgemisch wurde durch Destillieren von nicht umgesetztem Isobuten weitgehend befreit und hatte die in der Tabelle angegebene Zusammensetzung.

### Tabelle

| Komponente | Gew.-% |
|---|---|
| Isobuten | 0,7 |
| tert.-Butylalkohol | 27,2 |
| aec.-Butylalkohol | 33,0 |
| Isobutenoligomere | 1,8 |
| sec.-Butyl-tert.-butylether | 37,0 |
| Wasser | 0,3 |

## Patentansprüche

1. Verfahren zur Herstellung eines gegebenenfalls sec.-Butylalkohol enthaltendes Gemisches von sec.-Butyl-tert.-butylether und tert.-Butylalkohol aus Butan, dadurch gekennzeichnet, dass n-Butan partiell isomerisiert wird, die Butane katalytisch dehydriert werden, Butadien selektiv unter Umwandlung von Buten-1 zu Buten-2 hydriert, das im Dehydrierungsreaktionsgemisch enthaltene Isobuten mit rückgeführtem sec.-Butylalkohol-Wassergemisch unter Bildung von sec.-Butyl-tert.-butylether und tert.-Butylalkohol umgesetzt, Buten-2 aus dem nicht umgesetzten, vorwiegend Buten-2 und Butan enthaltenden Kohlenwasserstoffgemisch mit Wasser zu sec.-Butylalkohol-Wassergemisch umgewandelt und dieses in die Verätherung zurückgeführt wird.

2. Verfahren nach Anspruch 1, dadurch gekennzeichnet, dass Isobutan nach der Verätherung und nicht umgesetzte Kohlenwasserstoffe nach der Hydratisierung mit dem Isomerisat dehydriert werden.

3. Verfahren nach Anspruch 1 oder 2, dadurch gekennzeichnet, dass ein sec.-Butylalkohol-Wassergemisch mit flüssigem Isobuten bei Temperaturen von 20-150°C in Gegenwart saurer Katalysatoren zu tert.-Butylalkohol und sec.-Butyl-tert.-butylether umgesetzt wird, wobei eine solche Isobutenmenge eingesetzt wird, dass auf 1 Mol sec.-Butylalkohol 0,1-10 Mole Isobuten und auf 1 Mol Wasser 1-20 Mole Isobuten entfallen.

4. Verfahren nach einem der Ansprüche 1 bis 3, dadurch gekennzeichnet, dass durch Rektifizieren aus dem Reaktionsgemisch der Hydratisierung ein Wasser-sec.-Butylalkohol-Gemisch abgetrennt, aus diesem durch Behandeln mit einer Isobuten enthaltenden $C_4$-Fraktion ein sec.-Butylalkohol und Isobuten sowie Wasser enthaltendes Gemisch extraktiv abgetrennt und der Verätherung zugeführt wird, während die von sec.-Butylalkohol und Wasser befreite $C_4$-Fraktion in die Extraktion und die wässrige Phase aus der Extraktion in die Hydratisierung zurückgeführt werden.

5. Verfahren nach einem der Ansprüche 1 bis 3, dadurch gekennzeichnet, dass aus dem Reaktionsgemisch der Hydratisierung durch Behandlung mit einem vorwiegend aus n-Butan und Buten-2 bestehenden Kohlenwasserstoffgemisch ein Wasser enthaltender sec.-Butylalkohol extraktiv abgetrennt und der Verätherung zugeführt wird, während das von sec.-Butylalkohol und Wasser befreite Kohlenwasserstoffgemisch anteilig in die Hydratisierung und die Extraktion, die wässrige Phase aus der Extraktion in die Hydratisierung zurückgeführt werden.

## Claims

1. Process for producing a mixture of tert-butanol and sec-butyl tert-butyl ether which mixture may contain sec-butanol, too from butane, characterized in that n-butane is partially isomerized, the butanes are catalytically dehydrogenated, butadiene is selectively hydrogenated with conversion of butene-1 to butene-2, the isobutene contained in the dehydrogenation reaction mixture is etherified with recycled sec-butanol to form sec-butyl tert-butyl ether and tert-butanol, butene-2 from the unreacted hydrocarbon mixture which contains primarily butene-2 and butane, is converted with water to sec-butanol and this is recycled to the etherification.

2. Process according to Claim 1, characterized in that isobutane after etherification, and unconverted hydrocarbons after the hydration are dehydrogenated together with the isomerisate.

3. Process according to Claim 1 or 2, characterized in that a mixture of sec-butanol and water is reacted with liquid isobutene at temperatures of 20 to 150°C in the presence of acid catalysts to form tert-butanol and sec-butyl tert-butyl ether, wherein the amount of isobutene added provides 0.1 to 10 moles of isobutene per 1 mole of sec-butanol and 1 to 20 moles of isobutene per 1 mole of water.

4. Process according to one of the Claims 1 to 3, characterized in that a mixture of water and sec-butanol is separated by fractionation from the hydration reaction mixture, by treatment with a $C_4$-fraction containing isobutene a mixture containing sec-butanol, isobutene and water is extractively separated from this mixture of water and sec-butanol and taken to the etherification, while the $C_4$-fraction freed from sec-butanol and water is recycled to the extraction and the aqueous phase from the extraction is recycled to the hydration.

5. Process according to one of the Claims 1 to 3, characterized in that sec-butanol containing water is extracted from the hydration reaction mixture by treatment with a hydrocarbon mixture consisting mainly of n-butane and butene-2 and is taken to the etherification, while the hydrocarbon mixture freed from sec-butanol and water is proportionally recycled to the hydration and to the extraction, and the aqueous phase from the extraction is recycled to the hydration.

## Revendications

1. Procédé pour la fabrication d'un mélange, contenant éventuellement de l'alcool butylique sec., de l'éther de butyle-tert. et de butyle sec., et d'alcool butylique tert., à partir de butane, caractérisé en ce que l'on isomérise partiellement le n-butane, déshydrogénise catalytiquement les butanes, hydrogénise sélectivement le butadiène avec transformation du butène-1 en butène-2, fait réagir l'isobutène contenu dans le mélange réactionnel de la déshydrogénation avec un mélange recyclé d'alcool butylique sec. et d'eau, avec formation d'éther de butyle-tert. et de butyle sec. et d'alcool butylique tert., transforme le butène-2 du mélange d'hydrocarbures contenant principalement du butène-2 et du butane, avec de l'eau, en un mélange d'alcool butylique sec. et d'eau, et retourne ce dernier à l'éthérification.

2. Procédé suivant la revendication 1, caractérisé en ce que l'isobutane, après l'éthérification, et les hydrocarbures non réagis, après l'hydratation, sont déshydrogénés, en commun avec l'isomérisat.

3. Procédé suivant l'une des revendications 1 et 2, caractérisé en ce qu'on fait réagir un mélange d'alcool butylique sec. et d'eau avec de l'isobutène liquide, à des températures de 20 à 150°C, en présence de catalyseurs acides, en alcool butylique tert. et enéther butyle tert. et de butyle sec., la quantité d'isobutène mise en oeuvre étant telle que l'on trouve, pour 1 mole d'alcool butylique sec. 0,1 à 10 moles d'isobutène, et pour une mole d'eau, 1 à 20 moles d'isobutène.

4. Procédé suivant l'une quelconque des revendications 1 à 3, caractérisé en ce que, par rectification du mélange réactionnel de l'hydratation, on sépare un mélange eau-alcool butylique sec. d'où on sépare, par extraction, par traitement avec une fraction en $C_4$ contenant de l'isobutène, un mélange d'alcool butylique sec. et d'isobutène, ainsi que d'eau et l'envoie à l'éthérification, pendant que la fraction en $C_4$ libérée de l'alcool butylique sec. et de l'eau est retournée à l'extraction, et la phase aqueuse de l'extraction est retournée à l'hydratation.

5. Procédé suivant l'une des revendications 1 à 3, caractérisé en ce que l'on sépare, du mélange réactionnel d'hydratation, par traitement avec un mélange d'hydrocarbures constitué principalement de n-butane et de butène-2, par extraction, un alcool butylique sec. contenant de l'eau, et l'envoie à l'éthérification, pendant que le mélange d'hydrocarbures libéré de l'alcool butylique sec. et de l'eau est retourné par parties à l'hydratation et à l'extraction, la phase aqueuse de l'extraction étant renvoyé à l'hydratation.